# NEW EUROPEAN PATENT SPECIFICATION

(11) **EP 0 677 284 B2**
(45) Date of publication and mention of the opposition decision: **10.08.2005**
(45) Mention of the grant of the patent: 09.06.1999
(21) Application number: 95103693.8
(22) Date of filing: 14.03.1995
(51) Int. Cl.: A61F 13/15

(54) **Absorbent article comprising containment flaps**
Saugfähiger Artikel mit Aufnahmelaschen
Article absorbant pourvu de rabats de retention

(30) Priority: 14.03.1994 US 213338
(43) Date of publication of application: 18.10.1995
(73) Proprietor: KIMBERLY-CLARK WORLDWIDE, INC., Neenah, Wisconsin 54956 (US)
(72) Inventor: Kielpikowski, David Peter, Appleton, WI 54915 (US)
(74) Representative: Davies, Christopher Robert

(56) References cited:
- EP-A- 0 243 013
- EP-A- 0 246 858
- EP-A- 0 263 720
- EP-A- 0 300 615
- EP-A- 0 409 149
- EP-A- 0 411 287
- EP-A- 0 565 058
- EP-A- 0 566 150
- EP-A- 0 567 105
- EP-A- 0 568 085
- EP-A- 0 585 085
- FR-A- 2 532 337
- GB-A- 2 270 247
- US-A- 5 064 489

## Description

The present invention relates to containment flaps, and to absorbent articles comprising these containment flaps.

Various containment flaps are known for use in connection with absorbent articles such as infant diapers, training pants, adult incontinence products and the like. Such containment flaps are generally employed along the longitudinal sides of an absorbent article such as a diaper. The containment flaps are generally thought to be particularly well suited for the containment of fecal matter and to prevent the lateral flow of liquid waste until such time as the liquid waste can be absorbed by the absorbent article.

Containment flaps are often used in connection with elasticized leg cuffs located at the leg openings of the absorbent article which leg cuffs perform an additional gasketing function.

Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. Typically, the containment flaps are formed by folding the material from which the containment flap is to be formed in a longitudinal direction such that the fold line defines the distal edge of the containment flap with the opposite longitudinal ends of the material forming the proximal edge of the containment flap. In this manner, the elastic member is encased by the folded edge of the material such that it cannot contact the body of a wearer during use.

In order to allow the elastic material to gather the containment flap and assist in maintaining it in an upright position, the elastic material is generally adhesively attached to the containment flap. Adhesive attachment of the elastic member to the containment flap results in a certain amount of the elastic nature of the elastic member being destroyed. Specifically, at the points where the elastic member is adhesively attached to the containment flap, the elastic member is no longer capable of being stretched. That is, it is no longer "elastic". Moreover, the use of adhesives to attach the elastic material, or in joining the two layers of material, has been found, in some instances, to undesirably affect the stiffness of the containment flap.

Finally, known methods of constructing containment flaps tend to be relatively complex such that they may be a rate-limiting factor in certain continuous processes for producing absorbent articles.

EP-A-0 411 287 describes a garment with a waste containment pocket defined by spaced elasticized flaps and a method for producing the same. The flaps are formed by placing two elastic members onto a layer and applying an adhesive.

EP-A-0 568 085 describes a containment flap as mentioned in the preamble of claim 1 and a method in which the two elastic elements are provided on a sheet and adhered thereto by use of an adhesive.
EP-A-0 300 615 relates to a disposable diaper comprising elasticized leg cuffs. The leg cuffs are elasticized by positioning elastic members between first and second layers which layers are bonded to each other by bond segments.
EP-A-0 409 149 relates to an absorbent article comprising an absorbent core interposed between a liquid-impermeable backsheet and a liquid-permeable topsheet, and further comprising first and second flaps which extend laterally from the edges of the absorbent core.

Accordingly, it is the object of the present invention to provide an absorbent article employing a containment flap which is generally flexible and relatively easy to manufacture and which may be more economically manufactured and produced than certain known containment flaps.

This object is solved by the absorbent article according to independent claim 1. Further advantageous features, aspects and details of the present invention are evident from the dependent claims, the description and the drawings.

In one aspect, the present invention provides an absorbent article having a front portion, a rear portion, and a crotch portion connecting the front and rear portions. The crotch portion has opposite longitudinal side portions. The absorbent article comprises a liquid-permeable bodyside liner, an outer cover, and an absorbent core located between the bodyside liner and the outer cover. A pair of containment flaps extends longitudinally from the front portion of the absorbent article to the rear portion. The containment flaps have a proximal edge and a distal edge opposite the proximal edge. The proximal edge is joined to the bodyside liner in the crotch portion and in the front and rear portions. The containment flaps comprise a first layer of heat-fusible material, a second layer of heat-fusible material, and an elastic member located between the first and second layers of heat-fusible material adjacent the distal edge.

A first pattern of thermal bonds joins said first and second layers together. The first pattern of thermal bonds is located between the elastic member and the distal edge such that the elastic member is prevented from contacting the skin of a wearer.

A pattern of intermittent, thermal bonds joins said first and second layers together and intermittently, thermally bonds said elastic member to said first and second layer.

In one embodiment of the present invention, said first and second layers are formed from a single, integral piece of material which is folded upon itself. A pattern of intermittent, thermal bonds joins said first and second layers together and intermittently, thermally bonds said elastic member to said first and second layers.
Fig. 1 illustrates a partial cutaway plan view of a containment flap which is not according to the present invention.
Fig. 2 illustrates cutaway plan view of a containment flap which is not according to the present invention.
Fig. 3 illustrates a partial cutaway plan view of an embodiment of a containment flap for use in articles according to the present invention.
Fig. 4 illustrates a partial cutaway plan view of a containment flap which is not according to the present invention.
Fig. 5 illustrates a top plan view of a diaper according to the present invention.
Fig. 6 illustrates a cross-sectional perspective view taken along fine 6-6 of Fig. 5.
Fig. 7 illustrates a schematic illustration of a method for producing containment flaps for use in absorbent articles according to the present invention.
Fig. 8 illustrates a top plan view of the containment flap as it is produced according to the method of Fig. 7.
Fig. 9 illustrates a cross-sectional view taken along line 9-9 of Fig. 8.
Fig. 10 is a cross-sectional view of the containment flap illustrated in Fig. 9 in position on a diaper.

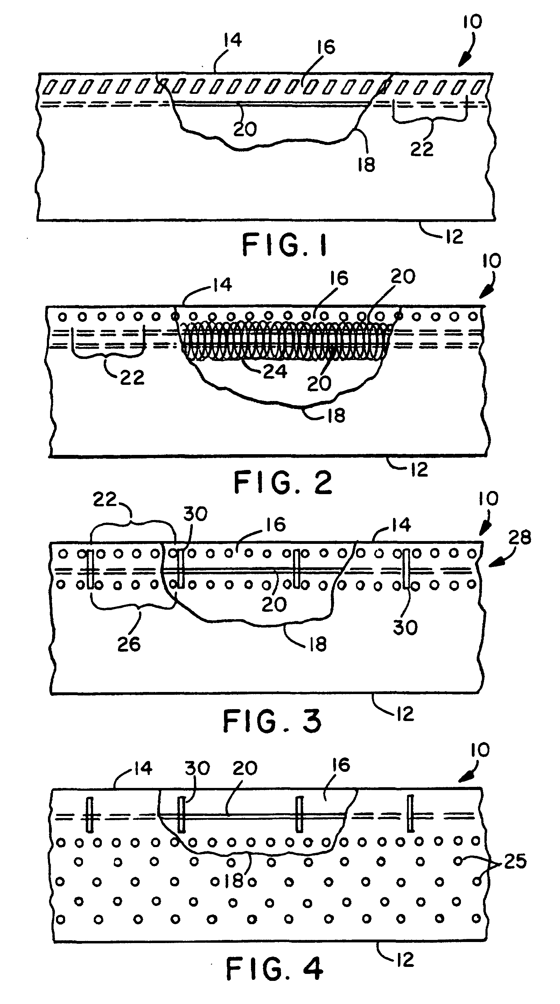

The present invention provides an absorbent article having a pair of containment flaps. The present invention can best be understood by reference to the drawings in which like numerals represent like elements. Figures 1, 2 and 4 illustrate containment flaps which are not in accordance with the present invention, but facilitate understanding of certain aspects of the present invention. Figure 3 shows an containment flap for use in an absorbent article in accordance with an embodiment of the present invention. Fig. 1 illustrates a partial cutaway top plan view of a containment flap. In Fig. 1, containment flap 10 defines a proximal edge 12, adapted to be joined to an absorbent article, and a distal edge 14 opposite the proximal edge 12. Containment flap 10 comprises a first layer of heat-fusible material 16, a second layer of heat-fusible material 18, and an elastic member 20 located between the first layer 16 and the second layer 18. The elastic member 20 is positioned adjacent distal edge 14.

A first pattern of thermal bonds 22 joins the first and second layers of heat-fusible material together. The first pattern of thermal bonds 22 is located between the elastic member 20 and the distal edge 14 such that the elastic member 20 is prevented from contacting the skin of a wearer when the containment flap is used on an absorbent article.

A containment flap 10 is illustrated in Fig. 2. Again, the containment flap defines a proximal edge 12 and a distal edge 14 located opposite the proximal edge 12. The containment flap is formed from a first layer of heat-fusible material 16 and a second layer of heat-fusible material 18. In this embodiment, two elastic members 20 are located between the first and second layers adjacent the distal edge 14. The two elastic members 20 are adhesively attached to the first layer 16 by adhesive 24.

Fig. 3 illustrates a partial cutaway plan view of an embodiment of a containment flap for use in absorbent articles according to the present invention. In the embodiment illustrated in Fig. 3, containment flap 10 includes intermittent, thermal bonds 30 which join said first and second layers together and intermittently, thermally bond said elastic members to said first and/or second layers. Containment flap 10 includes the first pattern of thermal bonds 22 and may further comprise a second pattern of thermal bonds 26 bonding the first and second layers of heat-fusible material together. The second pattern of thermal bonds is located between the elastic member 20 and the proximal edge 12. In this way, a channel 28 is formed between the first and second layers of heat-fusible material. The elastic member 20 is located in channel 28.

Fig. 4 illustrates a partial cutaway plan view of a containment flap which is not according to the present invention as it does not include the first pattern of thermal bonds 22 between the elastic member 20 and the distal edge 14. In the embodiment illustrated in Fig. 4, containment flap 10 is formed from a single, integral piece of material which is folded to define distal edge 14, the first layer of heat-fusible material 16 and the second layer of heat-fusible material 18. Elastic member 20 is located between the first and second layers of heat-fusible material. Intermittent, thermal bonds 30 join said first and second layers together and intermittently, thermally bond said elastic member to said first and/or second layers. The containment flap 10 may also comprise thermal bonds 25 which further bond said first and second layers together.

The containment flaps illustrated in Figs. 1-4 are adapted for use on absorbent articles. Typically, the containment flaps will be attached to the absorbent article at or adjacent proximal edge 12. At least a portion of the distal edge 14 will be unattached to the absorbent article such that the containment flap 10 provides a barrier to the lateral flow of waste material.

A wide range of materials are suitable for use as the first and second layers of heat-fusible material. A material will be considered to be heat fusible when an applied thermal energy causes it to soften or melt such that it can be joined to another material. As used herein, reference to "thermal bonds", "thermal bonding", "thermally bonded", "thermally attached" and the like refer to bonding caused by the application of thermal energies, such as heat, ultrasonic energy, infra red energy, and the like.

Specific examples of materials suitable for use as the first and second layers of heat-fusible material include nonwoven materials, such as spunbond or meltblown thermoplastic polymers, such as polyolefins; bonded carded webs; film materials, such as polyolefin, ethylene vinyl acetate, ethyl methacrylate, and polyester films; foam materials, such as polyolefin foams; woven materials, such as woven polypropylene, polyethylene or polyester fabrics; and composites and laminates of the above nonwoven, film, foam, and woven materials. In a specific embodiment of the present invention, the first and second heat-fusible materials are non-integrally formed in the namer illustrated by the arrangement of Figure 1. That is, the first and second layers of heat-fusible material represent separate elements which are not joined other than by thermal, adhesive, or similar attaching techniques. Specifically, the first and second layers are not formed from an integral piece of material through a folding process. In another specific embodiment of the present invention, the first and second heat-fusible layers are integrally formed in the namer illustrated by the arrangement of figure 4. That is, the first and second layers are formed from a single, integral piece of material through a folding process. If the containment flap of Fig. 3 is formed from a single, integral piece of material, the presence of thermal bonds 22 causes the distal edge to "blouse" when in use on an absorbent product in accordance with the present invention.

In another specific embodiment, the first and second layers of heat-fusible materials are formed from a nonwoven material such as a spunbond or meltblown polyethylene or polypropylene material. When it is desired to provide a containment flap which is generally liquid pervious, the spunbond material will suitably be treated with a surfactant to render it generally hydrophilic. Alternatively, one of the first or second layers of heat-fusible material may be treated with a surfactant while the other layer is treated to a lesser extent with a surfactant or is not treated with a surfactant. In this case, a containment flap which is less pervious to liquid will be produced. Still further, if it desired to produce a containment flap which is generally liquid impervious, one of the first or second layers of heat-fusible material can comprise a liquid-impervious film, such as a polyolefin film.

In one specific embodiment, the first layer of heat-fusible material comprises a polypropylene spunbond material having a basis weight of about 14.0 grams per square meter (about 0.4 ounce per square yard) and being formed from fibers having a tex (denier) of about 0.27-0.43 (about 2.5-3.8 d), which material is treated with a surfactant commercially available from Rohm and Haas Company, Philadelphia, PA, under the trade designation Triton X-102, at an add-on rate of about 0.3 weight percent. The second layer of heat-fusible material comprises a spunbond/meltblown composite formed from polypropylene and having a basis weight of about 28.0 grams per square meter (about 0.8 ounce per square yard) and comprising from about 50 percent spunbond material and about 50 percent meltblown material.

The elastic member 20 may comprise any elastomeric material capable of being elongated at least about 50 percent, alternately at least about 250 percent, alternately at least about 350 percent, and capable of recovering to a length within at least about 75 percent. more particularly, at least about 50 percent of its elongated length (original length plus elongation). The elastic member may be in the form of ribbons, individual strands, or other configurations. In one embodiment, the elastic member is in the form of individual elastomeric threads of elastomeric material. As described above, the containment flaps may comprise a single, elastic member or two or more elastic members. In one specific embodiment, the elastic member comprises a 470 decitex Lycra® thread commercially available from E. I. Dupont de Nemours and Co. Alternatively, the elastic member 20 can be composed of a thermoplastic elastomer or a natural or synthetic rubber commercially available from J.P.S. Elastomerics Corp. The elastic member 20 can also be composed of a heat-activatable elastic material such as Pebax®, commercially available from Atochem, Inc., which can be activated with heat treatment after the association with the containment flap.

In the containment flaps illustrated in Figs. 3 and 4, the elastic member is intermittently, thermally attached to the first and/or second layer of heat-fusible material. Specifically, intermittent, thermal bonds 30 soften or partially melt the first and/or second layers 16 and 18 so that they become thermally bonded. The elastic member 20 is trapped by the softened or melted material forming the first and/or second layers 16 and 18. It is generally desired that elastic member 20 not be melted or decomposed in the area of intermittent, thermal bond 30 so that the integrity of the elastic member 20 is retained along the length of the containment flap. Accordingly, the melting point or decomposition point of the elastic member 20 should be greater than the softening and/or melting point of the first and/or second layers of heat-fusible materials. Specifically, the melting point or decomposition point of the elastic member 20 may be at least about 5 degrees Centigrade, more specifically, at least about 10 degrees Centigrade greater than the melting point of the first and/or second layers of heat-fusible material. In this manner, the first and second layers of heat-fusible material may be melt bonded together so as to entrap and hold the elastic member 20 without causing the elastic member 20 to melt or decompose.

In the containment flap illustrated in Fig. 2. two elastic members 20 are shown as being adhesively attached to the first layer 16 by adhesive 24. Those skilled in the art will recognize that a wide variety of adhesive materials are suitable for use as adhesive 24. Specifically, the adhesive 24 may comprise a hot melt adhesive, a pressure-sensitive adhesive, latex adhesive, and the like. In one specific embodiment, the adhesive 24 comprises a hot melt adhesive commercially available from Findley Adhesives, Inc. under the trade designation H2096. The adhesive 24 may be applied in a wide variety of patterns known to those skilled in the art. Specifically, the adhesive 24 may be applied in swirls, strands, strips, bars, spots, and the like, and may be applied via slot coating, spraying, printing, and the like. Those skilled in the art will recognize that, at the points at which elastic member 20 is adhered to the first layer of heat-fusible material, the elastic nature of the elastic member is destroyed. That is, the elastic cannot be stretched over that length of the elastic member 20 which is adhesively attached to the first substrate. In order to compensate for the partial deadening of the elastic nature of the elastic member 20, it is generally desirable to use an elastic member having sufficient contractive forces to compensate for the partial loss of elastic nature caused by adhesive 24.

Similarly, in the containment flaps illustrated in Figs. 3 and 4, the elastic nature of the elastic member 20 is destroyed in the area of intermittent, thermal bonds 30. However, the intermittent, thermal bonds 30 generally provide a stronger attachment to the first layer 16 than do the adhesive bonds created by adhesive 24 illustrated in Fig. 2. Thus, it is possible to space the thermal bonds 30 a greater distance apart than is generally done with the use of adhesives, such as illustrated in Fig. 2.

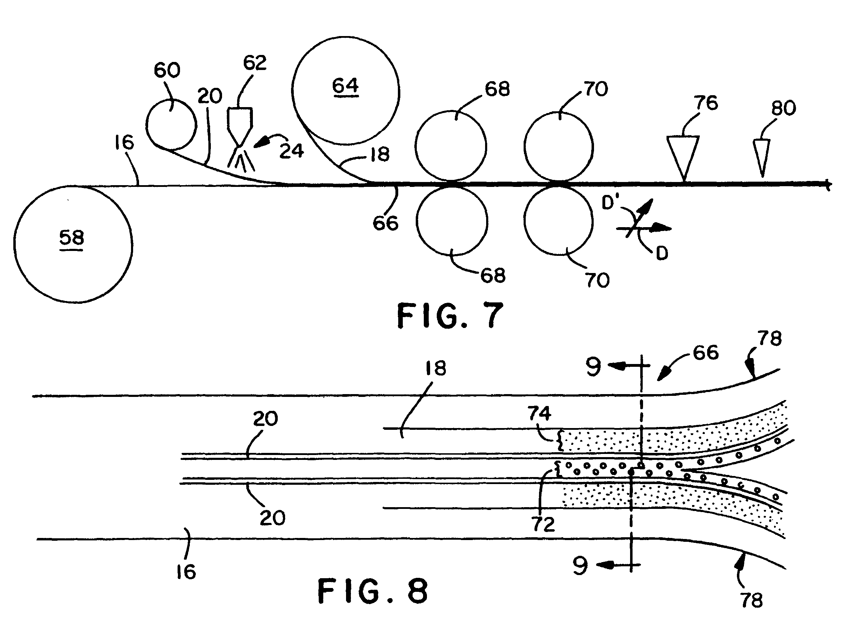

The thermal bonds 30 are intermittently spaced from about 10 cm (about 4 inches) to about 1 cm (about 0.4 inch) apart. Further, the thermal bonds 30 may be placed in a pattern of varying spacing allowing for increased bonding or decreased elastic retraction along the length of the flap. When the flaps are produced in a process, such as that illustrated below in connection with Figs. 7 and 8, the intermittent, thermal bonds may be located so that, when individual flaps are formed (such as by the cuts made by cutter 80 described in connection with Fig. 7 below), the elastic member retracts from the longitudinal ends of the flaps to the thermal bond 30. This creates an area of the containment flap which is free of elastication. This area which is free of elastication is suitably at least about 2.54 cm (about 1 inch) in length, particularly about 2.54 cm (about 1 inch) to about 12.7 cm (about 5 inches) in length.

Due to the greater spacing between thermal bonds 30, it is generally possible to use an elastic member having less contractive forces in the containment flaps illustrated in Figs. 3 and 4 than in that containment flap illustrated in Fig. 2. Alternatively it may be possible to use a single, elastic member illustrated in Figs. 3 and 4. while it may be desirable to use at least two of the same elastic members in the containment flap illustrated in Fig. 2.

The first pattern of thermal bonding illustrated in Figs. 1-3 serves to provide a seal along distal edge 14 such that the elastic member 20 cannot contact the skin of a wearer during use. In the past, distal edge 14 was often formed by folding. In this manner, elastic member 20 was prevented from contacting the skin of a wearer during use. However, the process to form such a folded distal edge was, in certain instances, undesirably complex. The use of the first pattern of thermal bonds to join the first and second layers together, such that the elastic member 20 is prevented from contacting the skin of a wearer, allows for the more efficient production of containment flaps.

Further, when the first pattern of thermal bonds joins first and second nonintegral layers together, it is possible to form shaped or curved containment flaps. Specifically, the distal edge of the containment flaps can be defined in a curved pattern, relative to the proximal edge, by the first pattern of thermal bonds. Excess material beyond the distal edge of the containment flap (as defined by the first pattern of thermal bonds) can then be trimmed and removed. In this manner, a containment flap, having different distances between its proximal and distal edges along its length, can be defined.

In the containment flaps illustrated in Figs. 3 and 4, use of the intermittent, thermal bonds 30 to join the elastic member 20 to the first and second layers can result in the formation of a containment flap that does not require the presence of any adhesive. This allows for the production of a containment flap with improved softness. That is, the presence of adhesive introduces a degree of stiffness to the containment flap that can be avoided through the sole use of thermal bonds.

When it is desired to minimize the use of thermal bonds in the containment flap, it is possible to form the flap generally illustrated in Fig. 4 which is not in accordance with the invention.

Those skilled in the art will recognize that the first and second patterns of thermal bonds can be formed in a wide variety of ways. Specifically, the first pattern of thermal bonds can be formed by ultrasonic bonding, heat bonding, infrared bonding, and the like. Moreover, the pattern may be formed of a wide variety of geometric shapes, such as squares, circles, dots, ellipses, and the like. The first pattern of thermal bonds is generally formed such that the thermal bonds join, via melt bonding, the first and second layers together along at least about 10, more particularly at least about 30, and more particularly at least about 50 percent of the length of the containment flap.

The second pattern of thermal bonds illustrated in Fig. 3 may be the same as the first pattern of thermal bonds or may be different. Specifically, it is believed that the second pattern of thermal bonds may comprise individual thermal bonds which are spaced further apart and thereby comprise a lower percent of the length of the containment flap than the first pattern of thermal bonds. This is because it is generally not as critical to prevent the elastic member from passing outside the second pattern of thermal bonds, as this would not cause the elastic member to contact the skin of a wearer.

Any method of forming the first and second patterns of thermal bonds is believed suitable for use in the present invention.

The thermal bonds 25 illustrated in Fig. 4 may be formed by any method and in any shape suitable for use in forming the first and second patterns of thermal bonds.

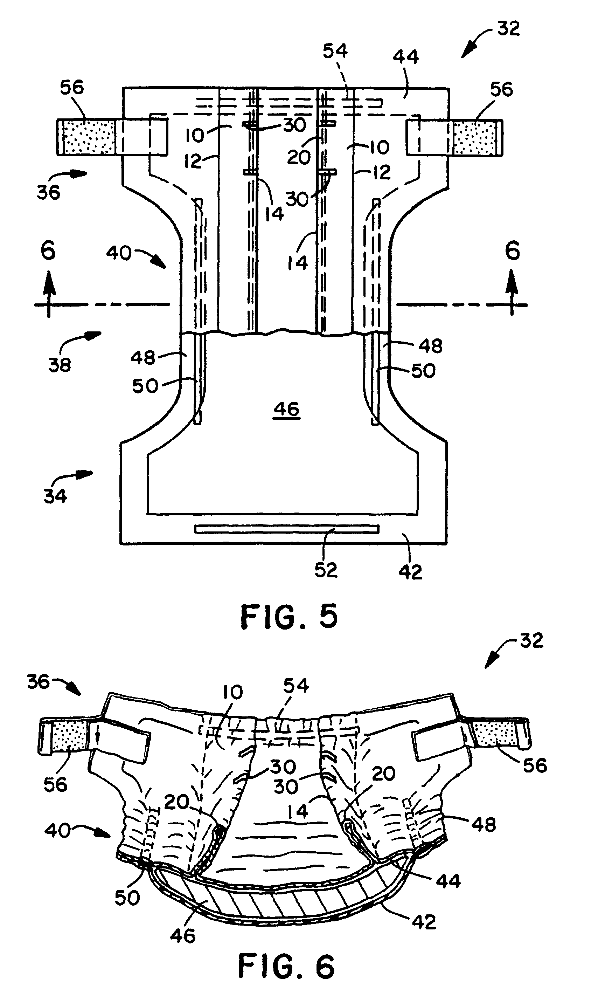

Figs. 5 and 6 illustrate a diaper according to the present invention. While the specific embodiments of the present invention will be described in terms of a disposable infant diaper adapted to be worn by infants about the lower torso, it is to be understood that the present invention is equally applicable to other absorbent articles, such as adult incontinence garments, training pants, feminine care products, and the like. With reference to Figs. 5 and 6, diaper 32 defines a front portion 34, a rear portion 36, and a crotch portion 38 connecting the front and rear portions. The crotch portion has opposite longitudinal side portions 40. Specifically, diaper 32 comprises an outer cover 42, a bodyside liner 44, and an absorbent core 46 located between the bodyside liner 44 and the outer cover 42. When used herein, reference to a front portion refers to that part of the diaper which is generally located on the front of an infant when in use. Reference to the rear portion refers to the portion of the diaper generally located at the rear of the infant when in use, and reference to the crotch portion refers to that portion which is generally located between the legs of an infant when in use.

The crotch portion 38 has opposite longitudinal side portions 40 which comprise a pair of elasticized, longitudinally-extending leg cuffs 48. The leg cuffs 48 are generally adapted to fit about the legs of a wearer when in use and serve as a mechanical barrier to the lateral flow of body exudates. Leg cuffs 48 are elasticized by leg elastics 50. Diaper 32 further comprises front waist elastic 52 and rear waist elastic 54. The rear portion 36 of diaper 32 also comprises fastening means, such as tape fasteners 56. The tape fasteners 56 are intended to hold the diaper 32 about the waist of an infant when in use.

Further, diaper 32 comprises containment flaps 10 such as those illustrated in Figs. 3: In the specific embodiment illustrated in Figs. 5 and 6, the diaper 32 comprises a containment flap such as that illustrated in Fig. 4 and is therefore not in accordance with the invention. The containment flap 10 is attached to bodyside liner 44 along proximal edge 12 in the front, rear, and crotch portion of the diaper 32. The distal edge 14 of containment flap 10 is adhered to the bodyside liner 44 of diaper 32 in at least a portion of the front and rear portion of diaper 32. In this way, elastic members 20 function to maintain the distal edge of containment flap 10 in an upright condition in the crotch portion of diaper 32 when in use on a wearer.

Bodyside liner 44 suitably presents a body-facing surface which is compliant, soft feeling and non-irritating to the wearer's skin. Further, bodyside liner 44 may be less hydrophilic than the absorbent core 46, to present a relatively dry surface to the wearer, and is sufficiently porous to be liquid permeable, permitting liquid to readily penetrate through its thickness. A suitable bodyside liner may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. Bodyside liner 44 is suitably employed to help isolate the wearer's skin from liquids held in absorbent core 46.

Various woven and nonwoven fabrics can be used for bodyside liner 44. For example, the bodyside liner may be composed of a meltblown or spunbonded web of polyolefin fibers. The bodyside liner may also be a bonded-carded web composed of natural and/or synthetic fibers. The bodyside liner may be composed of a substantially hydrophobic material, and the hydrophobic material may, optionally, be treated with a surfactant, or otherwise processed, to impart a desired level of wettability and hydrophilicity. In a particular embodiment of the present invention, the bodyside liner 44 comprises a nonwoven, spunbond, polypropylene fabric composed of about 0.31-0.36 tex (about 2.8-3.2 denier) fibers formed into a web having a basis weight of about 22 grams per square meter and a density of about 0.06 gram per cubic centimeter. The fabric is surface treated with about 0.28 weight percent of a surfactant commercially available from Rohm and Haas Co. under the trade designation Triton X-102.

The outer cover 42 may suitably be composed of a material which is either liquid permeable or liquid impermeable. It is generally preferred that the outer cover 42 be formed from a material which is substantially impermeable to liquids. For example, a typical outer cover can be manufactured from a thin plastic film or other flexible liquid-impermeable material. For example, the outer cover 42 may be formed from a polyethylene film having a thickness of from about 0.012 mm (0.5 mil) to about 0.051 mm (2.0 mils). If it is desired to present the outer cover 42 with a more clothlike feeling, the outer cover 42 may comprise a polyethylene film having laminated to the outer surface thereof a nonwoven web, such as a spunbond web of polyolefin fibers. For example, a polyethylene film having a thickness of about 0.015 mm (0.6 mil) may have thermally laminated thereto a spunbond web of polyolefin fibers, which fibers have a thickness of about 0.16 to 0.28 tex (about 1.5 to 2.5 denier) per filament, which nonwoven web has a basis weight of about 24 grams per square meter (0.7 ounce per square yard). Methods of forming such clothlike outer covers are known to those skilled in the art.

Further, the outer cover may be formed of a woven or nonwoven fibrous web layer which has been totally or partially constructed or treated to impart a desired level of liquid impermeability to selected regions that are adjacent or proximate the absorbent core 46. Still further, the outer cover 42 may optionally be composed of a micro-porous "breathable" material which permits vapors to escape from the absorbent core 46 while still preventing liquid exudates from passing through the outer cover 42.

The absorbent core 46 suitably comprises a matrix of hydrophilic fibers, such as a web of cellulosic fluff, mixed with particles of a high-absorbency material commonly known as superabsorbent material. In a particular embodiment, absorbent core 46 comprises a mixture of superabsorbent hydrogel-forming particles and wood pulp fluff. The wood pulp fluff may be exchanged with synthetic, polymeric, meltblown fibers or with a combination of meltblown fibers and natural fibers. The superabsorbent particles may be substantially homogeneously mixed with the hydrophilic fibers or may be non-uniformly mixed.

Alternatively, the absorbent core may comprise a laminate of fibrous webs and superabsorbent material or other suitable means of maintaining a superabsorbent material in a localized area.

The absorbent core 46 may have any of a number of shapes. For example, the absorbent core may be rectangular, I-shaped or T-shaped. It is generally preferred that the absorbent core be narrower in the crotch portion than the rear or front portion.

The high-absorbency material can be selected from natural, synthetic and modified natural polymers and materials. The high-absorbency materials can be inorganic materials, such as silica gels, or organic compounds, such as crosslinked polymers. The term "crosslinked" refers to any means for effectively rendering normally water-soluble materials substantially water insoluble but swellable. Such means can include, for example, physical entanglement, crystalline domains, covalent bonds, ionic complexes and associations, hydrophilic associations, such as hydrogen bonding, and hydrophobic associations or Van der Waals forces.

Examples of synthetic, polymeric, high-absorbency materials include the alkali metal and ammonium salts of poly(acrylic acid) and poly(methacrylic acid), poly(acrylamides), poly(vinyl ethers), maleic anhydride copolymers with vinyl .ethers and alpha-olefins, poly(vinyl pyrolidone), poly(vinyl morpholinone), poly(vinyl alcohol), and mixtures and copolymers thereof. Further polymers suitable for use in the absorbent core include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthum gum, locust bean gum, and the like. Mixtures of natural and wholly or partially synthetic absorbent polymers can also be useful in the present invention. Other suitable absorbent gelling materials are disclosed by Assarson et al. in U.S. Patent No. 3,902,236 issued August 26, 1975. Processes for preparing synthetic, absorbent gelling polymers are disclosed in U.S. Patent No. 4,076,663 issued February 28, 1978, to Masuda et al. and U.S. Patent No. 4,286,082 issued August 25, 1981, to Tsubakimoto et al.

The high-absorbency material may be in any of a wide variety of geometric forms. As a general rule, it is preferred that the high-absorbency material be in the form of discrete particles. However, the high-absorbency material may also be in the form of fibers, flakes, rods, spheres, needles, or the like.

As a general rule, the high-absorbency material is present in the absorbent core in an amount of from about 5 to about 100 weight percent based on total weight of the absorbent core.

The outer cover 42 and bodyside liner 44 are generally adhered to one another so as to form a pocket in which the absorbent core 46 is located. Thus, leg cuffs 48 are suitably formed by portions of the outer cover 42, and/or bodyside liner 44, which extend beyond the longitudinal sides of the absorbent core 46. Naturally, the leg cuffs 48 can also be formed from separate materials which are attached to the outer cover 42 and/or bodyside liner 44.

Leg cuffs 48 include leg elastics 50. Materials suitable for use in forming leg elastics 50 are known to those skilled in the art. Exemplary of such materials are strands or ribbons of a polymeric, elastomeric material which are adhered to the diaper at the leg cuff while in a stretched position, or which are attached to the diaper while the diaper is pleated, such that elastic constrictive forces are imparted to the leg cuff 48.

Similarly, waist elastics 52 and 54 and tape fasteners 56 suitable for use in the present invention are known to those skilled in the art.

A wide variety of other diaper configurations, as well as training pants, incontinence garments, and like configurations, are suitable for use in the present invention. Diapers suitable for use in the present invention are described in greater detail in commonly assigned U.S. Patent Application Serial No. 08/096,654 entitled "Thin Absorbent Article Having Rapid Uptake of Liquid" filed July 22, 1993, in the name of Hansen et al; and U.S. Patent No. 5,192,606 issued March 9, 1993, to Proxmire et al.

A method of making a containment flap for use on an absorbent article can best be understood by reference to Fig. 7 wherein a first layer of heat-fusible material 16 is provided from supply roll 58. The first layer of heat-fusible material 16 is traveling in a first direction D. Two elastic members 30 are supplied from supply roll 60 and are traveling in the first direction D and are attached to the first layer of heat-fusible material in a laterally-spaced relationship. This can be seen by reference to Fig. 8. In the illustrated arrangement elastic members 20 are attached to the first layer 16 by adhesive 24 applied from applicator 62 rather than using intermittent thermal bonds in accordance with the present invention. A second layer of heat-fusible material 18 is supplied from supply roll 64. The second layer of heat-fusible material is traveling in the first direction and is laid on top of the first layer 16 such that the elastic members 20 are located between the first and second layers. The composite 66, thus formed, then passes through nip roller 68. The composite 66 then passes through a thermal bonding means such as sonic bonder 70. As can be seen from reference to Fig. 8, the sonic bonder 70 thermally bonds the second layer of heat-fusible material to the first layer of heat-fusible material through thermal bonds 72 and 74. The composite 66 then passes through slitter 76 wherein composite 66 is slit in the first direction between elastic members 20 to form two slit composites 78. The two slit composites 78 are then cut in a second direction D', generally perpendicular to said first direction, by cutter 80 to form containment flaps.

As can be seen from reference to Figs. 7-9, the thermal bonds 72, after being slit, form the first pattern of thermal bonds 22 illustrated in Figs. 1-3, while the thermal bonds 74 form the second pattern of thermal bonds 26 illustrated in Fig. 3.

While Figs. 7-9 illustrate that elastic members 20 are adhesively attached to the first layer 16, it is to be understood that when making containment flaps for articles in accordance with the invention applicator 62 may be removed from the process, such that elastic members 20 are merely laid on top of the first layer 16 and that elastic members 20 are subsequently intermittently, thermally attached to the first layer 16 by sonic bonder 70 in accordance with the embodiment of the present invention illustrated in Fig. 3. Containment flaps produced by the above method are then suitably attached to an absorbent article as illustrated and described in connection with Figs. 5 and 6.

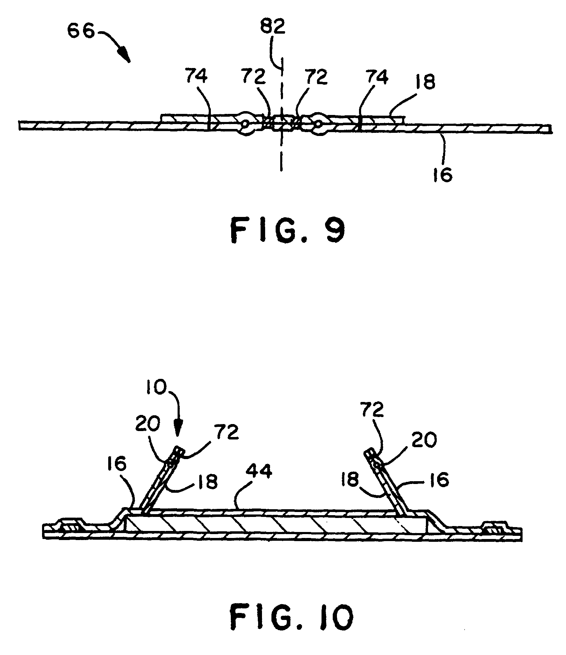

Fig. 9 is a cross section of composite 66 taken along line 9-9 of Fig. 8. Line 82 illustrates where the composite 66 will be slit by slitter 76.

As can be seen from reference to Fig. 9, the first layer of heat-fusible material is illustrated as being significantly wider than the second layer of heat-fusible material 18. In the illustrated embodiment, the first layer of heat-fusible material is a nonwoven material while the second layer of heat-fusible material 18 is a liquid-impervious material, such as a polyolefin film. Use of the containment flaps formed from the composite illustrated in Fig. 9 are shown in use in Fig. 10 which represents a cross-sectional view of an infant diaper similar to that illustrated in Fig. 5. As can be seen from reference to Fig. 10, the portion of the first layer 16 extending beyond the second layer 18 is used to form a portion of the bodyside liner 44 illustrated in Fig. 5. The second layer 18 is located on the interior surface of the containment flap 10 illustrated in Fig. 10. Thus, the bodyside liner 44 illustrated in Fig. 10 actually comprises a portion of bodyside liner 44 located between containment flaps 10 and that portion of first layer 16 of the containment flap 10 which extends beyond the width of the second layer 18.

The containment flaps illustrated in Fig. 4 can be formed according to the following method. A single, integral piece of heat-fusible material is provided. The heat-fusible material is folded to provide first and second layers of heat-fusible material. An elastic member is positioned between the first and second layers of heat-fusible material. The elastic member is then intermittently, thermally bonded to said first and second layers.

While the invention has been described in detail with respect to specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims.

## Claims

1. An absorbent article (32) having a front portion (34), a rear portion (36) and a crotch portion (38) connecting said front and rear portions (34, 36), said crotch portion (38) having opposite longitudinal side portions (40) comprising respective elasticized longitudinally-extending leg cuffs (48), said article (32) comprising:
a liquid-permeable bodyside liner (44);
an outer cover (42);
an absorbent core (46) located between said bodyside liner (44) and said outer cover (42); and
a pair of containment flaps (10) extending longitudinally from said front portion (34) to said rear portion (36), each said containment flap comprising a proximal edge (12) joined to said bodyside liner (44) in said crotch portion (38) and said front and rear portions (34, 36), and a distal edge (14) opposite said proximal edge (12), said containment flap (10) comprising:
a first layer (16) made of heat fusible material;
a second layer (18) made of heat fusible material;
at least one elastic member (20) located between said first and said second layers adjacent said distal edge (14);
**characterized in that**
a first pattern of bonds (22) joins said first and second layers (16, 18) together, said first pattern of bonds (22) being located between said elastic member (20) and said distal edge (14), and wherein
said first pattern of bonds (22) is provided by thermal bonds and said elastic member (20) is intermittently, thermally attached to said first and/or second layers (16, 18).

2. The absorbent article according to claim 1 wherein said first layer (16) comprises a nonwoven material.

3. The absorbent article according to claim 1 or 2 wherein said second layer (18) comprises a nonwoven material.

4. The absorbent article according to one of the preceding claims wherein said second layer (18) comprises a film.

5. The absorbent article according to one of the preceding claims wherein said containment flap (10) comprises two elastic members (20).

6. The absorbent article according to one of the preceding claims further comprising a second pattern of thermal bonds (26) located between said elastic member (20) and said proximal edge (12).

7. The absorbent article according to one of the preceding claims wherein said elastic member (20) has a melting or decomposition point greater than the melting point of said first layer (16) and/or said second layer (18).

8. The absorbent article according to one of the preceding claims wherein the melting or decomposition point of said elastic member (20) is at least 5°C greater than the melting point of said first layer (16) and/or said second layer (18).

9. The absorbent article according to one of the preceding claims wherein said containment flap (10) comprises a second pattern of thermal bonds (26) joining said first and second layers (16,18) together, said second pattern of thermal bonds (26) being located between said elastic member (20) and said proximal edge (12).

10. The absorbent article according to one of the preceding claims wherein said elastic member (20) is not melted or decomposed during the intermittent, thermal attachment of said elastic member (20) to said first and/or second layers (16,18).

11. The absorbent article according to one of claims 6 to 10 further comprising a second elastic member (20) located between said first and second patterns of thermal bonds (22, 26), said second elastic member (20) being intermittently, thermally attached to said first and second layer (16,18).

12. The absorbent article according to one of the preceding claims wherein said elastic member (20) comprises or is an elastomeric thread.

13. The absorbent article according to claim 11 wherein said elastomeric thread has a melting or decomposition point greater than the melting point of said first and second layers (16,18).

14. The absorbent article according to claim 6 wherein said containment flap (10) further comprises a second elastic member (20) located between said first and second pattern of thermal bonds (22,26).

15. The absorbent article according to one of the preceding claims wherein said first and second layers (16,18) are not integrally formed.

16. The absorbent article according to one of claims 1 to 14 wherein said first and second layers (16,18) of heat-fusible material are formed from a single, integral piece of material by folding.

## Patentansprüche

1. Saugfähiger Artikel (32) mit einem vorderen Bereich (34), einem hinteren Bereich (36) und einem Schrittbereich (38), welcher den vorderen und den hinteren Bereich (34, 36) verbindet, wobei der Schrittbereich (38) gegenüberliegende Längsseitenbereiche (40) aufweist, die jeweils elastische sich in Längsrichtung erstreckende Beinbündchen (48) umfassen, wobei der Artikel (32) umfasst:
eine körperseitige flüssigkeitsdurchlässige Ausfütterung (44);
eine äußere Abdeckung (42);
einen saugfähigen Kern (46), welcher zwischen der körperseitigen Ausfütterung (44) und der äußeren Abdeckung (42) angeordnet ist; und
ein Paar Rückhalteklappen (10), welche sich in Längsrichtung von dem vorderen Bereich (34) zum hinteren Bereich (36) erstrecken, wobei jede Rückhalteklappe eine proximale Kante (12), welche mit der körperseitigen Ausfütterung (44) in dem Schrittbereich (38) und dem vorderen und hinteren Bereich (34, 36) verbunden ist, und eine distale Kante (14) gegenüber der proximalen Kante (12) umfasst, wobei die Rückhalteklappe (10) umfasst:
eine erste Schicht (16) aus wärmeschmelzbarem Material hergestellt;
eine zweite Schicht (18) aus wärmeschmelzbarem Material hergestellt;
wenigstens ein elastisches Element (20), welches zwischen der ersten und der zweiten Schicht benachbart zu der distalen Kante (14) angeordnet ist;
**dadurch gekennzeichnet, dass**
ein erstes Muster von Bindungen (22) die erste und zweite Schicht (16, 18) miteinander verbindet, wobei das erste Muster von Bindungen (22) zwischen dem elastischen Element (20) und der distalen Kante (14) angeordnet ist, und wobei
das erste Muster von Bindungen (22) durch thermische Bindungen erzeugt ist und das elastische Element (20) intermittierend an der ersten und/oder zweiten Schicht (16, 18) thermisch befestigt ist.

2. Saugfähiger Artikel gemäß Anspruch 1, bei dem die erste Schicht (16) ein Vliesstoffmaterial umfasst.

3. Saugfähiger Artikel gemäß Anspruch 1 oder 2, bei dem die zweite Schicht (18) ein Vliesstoffmaterial umfasst.

4. Saugfähiger Artikel gemäß einem der vorhergehenden Ansprüche, bei dem die zweite Schicht (18) eine Folie umfasst.

5. Saugfähiger Artikel gemäß einem der vorhergehenden Ansprüche, bei dem die Rückhalteklappe (10) zwei elastische Elemente (20) umfasst.

6. Saugfähigher Artikel gemäß einem der vorhergehenden Ansprüche, der ferner ein zweites Muster von thermischen Bindungen (26) umfasst, welches zwischen dem elastischen Element (20) und der proximalen Kante (12) angeordnet ist.

7. Saugfähiger Artikel gemäß einem der vorhergehenden Ansprüche, bei dem das elastische Element (20) einen Schmelz- oder Zersetzungspunkt aufweist, der höher ist als der Schmelzpunkt der ersten Schicht (16) und/oder der zweiten Schicht (18).

8. Saugfähiger Artikel gemäß einem der vorhergehenden Ansprüche, bei dem der Schmelz- oder Zersetzungspunkt des elastischen Elements (20) wenigstens 5°C höher ist als der Schmelzpunkt der ersten Schicht (16) und/oder der zweiten Schicht (18).

9. Saugfähiger Artikel gemäß einem der vorhergehenden Ansprüche, bei dem die Rückhalteklappe (10) ein zweites Muster von thermischen Bindungen (26) umfasst, welche die erste und die zweite Schicht (16, 18) miteinander verbindet, wobei das zweite Muster von thermischen Bindungen (26) zwischen dem elastischen Element (20) und der proximalen Kante (12) angeordnet ist.

10. Saugfähiger Artikel gemäß einem der vorhergehenden Ansprüche, bei dem das elastische Element (20) während der intermittierenden, thermischen Befestigung des elastischen Elements (20) an der ersten und/oder der zweiten Schicht (16, 18) weder geschmolzen noch zersetzt wird.

11. Saugfähiger Artikel gemäß einem der Ansprüche 6 bis 10, ferner umfassend ein zweites elastisches Element (20), welches zwischen dem ersten und zweiten Muster von thermischen Bindungen (22, 26) angeordnet ist, wobei das zweite elastische Element (20) intermittierend an der ersten und zweiten Schicht (16, 18) thermisch befestigt ist.

12. Saugfähiger Artikel gemäß einem der vorhergehenden Ansprüche, bei dem das elastische Element (20) einen elastomeren Faden umfasst oder ein elastomerer Faden ist.

13. Saugfähiger Artikel gemäß Anspruch 11, bei dem der elastomere Faden einen Schmelz- oder Zersetzungspunkt aufweist, welcher höher ist als der Schmelzpunkt der ersten und zweiten Schicht (16, 18).

14. Saugfähiger Artikel gemäß Anspruch 6, bei dem die Rückhalteklappe (10) ferner ein zweites elastisches Element (20) umfasst, welches zwischen dem ersten und dem zweiten Muster von thermischen Bindungen (22, 26) angeordnet ist.

15. Saugfähiger Artikel gemäß einem der vorhergehenden Ansprüche, bei dem die erste und die zweite Schicht (16, 18) nicht integral ausgebildet sind.

16. Saugfähigher Artikel gemäß einem der Ansprüche 1 bis 14, bei dem die erste und die zweite Schicht (16, 18) aus wärmeschmelzbarem Material aus einem einzigen integralen Materialstück mittels Faltung gebildet sind.

## Revendications

1. Article absorbant (32) ayant une portion avant (34), une portion arrière (36) et une portion d'entrejambe (38) connectant lesdites portions avant et arrière (34, 36), ladite portion d'entrejambe (38) ayant des portions (40) latérales, longitudinales, opposées, comprenant des bordures de jambe (48), élastiquées, s'étendant longitudinalement, respectives, ledit article (32) comprenant :
une doublure côté corporel (44) perméable aux liquides ;
une feuille de couverture extérieure (42) ;
un noyau absorbant (46) situé entre ladite doublure côté corporel (44) et ladite feuille de couverture extérieure (42) ; et
une paire de volets de retenue (10) s'étendant longitudinalement depuis ladite portion avant (34) vers ladite portion arrière (36), chacun desdits volets de retenue comprenant un bord proximal (12) adapté à être réuni à ladite doublure côté corporel (44) dans ladite portion d'entrejambe (38) et lesdites portions avant et arrière (34, 36), et un bord distal (14) à l'opposé dudit bord proximal (12), ledit volet de retenue (10) comprenant :
une première couche (16) faite d'un matériau thermofusible ;
une deuxième couche (18) faite d'un matériau thermofusible ;
au moins un élément élastique (20) situé entre lesdites première et deuxième couches au voisinage dudit bord distal (14) ;
**caractérisé en ce que**
un premier motif de liaisons (22) réunit ensemble lesdites première et deuxième couches (16, 18), ledit premier motif de liaisons (22) étant situé entre ledit élément élastique (20) et ledit bord distal (14), et dans lequel
ledit premier motif de liaisons (22) est fourni par des liaisons thermiques et ledit élément élastique (20) est fixé thermiquement, par intermittence, auxdites première et/ou deuxième couche(s) (16, 18).

2. Article absorbant selon la revendication 1, dans lequel ladite première couche (16) comprend un matériau'non tissé.

3. Article absorbant selon la revendication 1 ou 2, dans lequel ladite deuxième couche (18) comprend un matériau non tissé.

4. Article absorbant selon l'une des revendications précédentes, dans lequel ladite deuxième couche (18) comprend un film.

5. Article absorbant selon l'une des revendications précédentes, dans lequel ledit volet de retenue (10) comprend deux éléments élastiques (20).

6. Article absorbant selon l'une des revendications précédentes, comprenant en outre un deuxième motif de liaisons thermiques (26) situé entre ledit élément élastique (20) et ledit bord proximal (12).

7. Article absorbant selon l'une des revendications précédentes, dans lequel ledit élément élastique (20) a un point de fusion ou de décomposition supérieur au point de fusion de ladite première couche (16) et/ou de ladite deuxième couche (18).

8. Article absorbant selon l'une des revendications précédentes, dans lequel le point de fusion ou de décomposition dudit élément élastique (20) est au moins 5°C supérieur au point de fusion de ladite première couche (16) et/ou de ladite deuxième couche (18).

9. Article absorbant selon l'une des revendications précédentes, dans lequel ledit volet de retenue (10) comprend un deuxième motif de liaisons thermiques (26) réunissant ensemble lesdites première et deuxième couches (16, 18), ledit deuxième motif de liaisons thermiques (26) étant situé entre ledit élément élastique (20) et ledit bord proximal (12).

10. Article absorbant selon l'une des revendications précédentes, dans lequel ledit élément élastique (20) n'est pas fondu ou décomposé au cours de la fixation thermique intermittente dudit élément élastique (20) auxdites première et/ou deuxième couches (16, 18).

11. Article absorbant selon l'une des revendications 6 à 10, comprenant en outre un deuxième élément élastique (20) situé entre lesdits premier et deuxième motifs de liaisons thermiques (22, 26), ledit deuxième élément élastique (20) étant fixé thermiquement, par intermittence, auxdites première et deuxième couches (16, 18).

12. Article absorbant selon l'une des revendications précédentes, dans lequel ledit élément élastique (20) comprend, ou est constitué par, un fil élastomère.

13. Article absorbant selon la revendication 11, dans lequel ledit fil élastomère a un point de fusion ou de décomposition supérieur au point de fusion desdites première et deuxième couches (16, 18).

14. Article absorbant selon la revendication 6, dans lequel ledit volet de retenue (10) comprend en outre un deuxième élément élastique (20) situé entre lesdits premier et deuxième motifs de liaisons thermiques (22, 26).

15. Article absorbant selon l'une des revendications précédentes, dans lequel lesdites première et deuxième couches (16, 18) ne sont pas d'un seul tenant.

16. Article absorbant selon l'une des revendications 1 à 14, dans lequel lesdites première et deuxième couches (16, 18) de matériau thermofusible sont formées, d'un seul tenant, à partir d'une unique pièce de matériau, par pliage.
